# EUROPEAN PATENT APPLICATION

(11) **EP 3 838 878 A1**
(43) Date of publication of application: **23.06.2021**
(21) Application number: 19306737.8
(22) Date of filing: 20.12.2019
(51) Int. Cl.: C07C 2/76, B01J 21/12, B01J 23/745, B01J 35/00, B01J 37/12

(54) **SILICON-BASED MATERIALS, THEIR PREPARATION AND APPLICATIONS**

(71) Applicant: TOTAL SE, 92400 Courbevoie (FR)
(72) Inventor: SACHS, Christoph, 78530 BUC (FR); VAN DAELE, Stijn, 1500 HALLE (BE); NESTERENKO, Nikolai, 1402 NIVELLES (BE)
(74) Representative: Lavoix

(57) **Abstract**

The present invention relates to a material comprising (i) an inner part comprising or consisting of bulk silicon, (ii) an outer part comprising or consisting of a silicon-based compound, said silicon-based compound comprising of silicon and a non-metal element, and (iii) clusters comprising or consisting of a transition metal.

The present invention relates to preparation and applications of said material.

## Description

### Technical field

The present invention concerns a new material, its preparation and applications, especially in the field of catalysis.

More particularly, the present invention relates to materials for catalyzing chemical reactions in different industries, especially in the oil, gas or chemical industry. The surface chemistry and morphology are important factors for the catalytic activity and the resulting product selectivity. The use of silicon offers an opportunity to control the distribution of active elements such Fe and other transition metals. Furthermore, novel routes for the synthesis of such materials are possible. The basic concept is that the silicon surface can be doped with a transition metal, followed by a conversion of the silicon surface into a silicon compound such as silicon oxide. The thermal and chemical stability of silicon as well as the low solubility and diffusivity of transition metals in silicon will confine these elements to single-atom sites, clusters or domains with high concentration.

### Background of the invention

Silicon is mainly known as an alloying element for the steel and aluminum industry or if highly purified as a material for the semiconductor and photovoltaic industry. However, silicon has other properties that makes it suitable for novel applications ranging from the biomedical field, energy storage to structural materials. It can also serve as a new material for catalysts which are an important group of materials for carrying out chemical reactions in many industries, especially the oil, gas and chemical industry. The surface chemistry and morphology are important factors for the catalytic activity and the product selectivity. The use of silicon offers an opportunity to control the distribution of active elements such Fe and other transition metals and novel routes for the synthesis of catalysts. The basic concept is that the silicon surface or bulk can be doped with a transition metal or other active elements, followed by a conversion of the silicon surface into a silicon compound such as silicon oxide. The thermal and chemical stability of silicon as well as the low solubility and diffusivity of other elements such as transition metals in silicon will confine the mobility of the elements. Thus, it is possible to generate active sites on the surface where catalytic reaction may take place, for example for gas conversion.

As catalytic activity and selectivity depend on the surface chemistry and morphology, it is generally beneficial to have (1) a proper surface area, typically from 10 to 100 m²/g, (2) sufficient temperature resistance, (3) proper density of active (catalytic) sites, and (4) a stable configuration.

Today, the synthesis of such materials often requires careful processing. According to the prior-art on catalysts, a new catalyst material for conversion of natural gas is Fe(c)SiO₂, which can be prepared by mixing silicon dioxide with iron, crushing (by ball milling) and undergoing a heat treatment at a high temperature of approximately 1700°C to provide the Fe(c)SiO₂ catalyst. A key characteristic of the obtained catalyst was that it contained single Fe-atom sites. It seems that catalyst sites below 5nm show better catalytic performance. A processing route is described by Guo et al. (Science, 2014, 344, 616-619). However, this kind of high temperature synthesis is rather challenging and the process window might be narrow, given the need to well control the nucleation rate and growth of Fe precipitates. From an industrial point of view, it would be desirable to have material synthesis routes that are stable, well reproducible and scalable at low cost.

### Aims of the invention

The present invention aims to provide a material that could be produced in a reliable manner in view of industrial technical needs, and in particular to provide a process for manufacturing a material in a reproducible way.

The present invention aims to provide such a material at acceptable industrial costs.

The present invention aims to provide a material that can be used as a catalyst overcoming the above technical difficulties.

In particular, the present invention aims to provide a catalyst for natural gas conversion.

The present invention aims to provide a catalyst with good catalytic properties (selectivity, conversion).

In particular, the present invention aims to provide a material with a functionalized surface, i.e. surface areas or clusters containing transition metals with a size of less than 5 nm.

### Detailed description of the invention

The present invention relates to a material comprising (i) an inner part comprising or consisting of bulk silicon, (ii) an outer part comprising or consisting of a silicon-based compound, said silicon-based compound comprising of silicon and a non-metal element, and (iii) clusters comprising or consisting of a transition metal.

According to inventors' knowledge, contrary to the present invention, no present technical art started from a silicon inner part (also called core, inner layer, matrix or substrate) to prepare a surface that is decorated with clusters containing transition metals.

It was hypothesized and proven by the present inventors that by preparing a material with a core or inner part of silicon instead of silicon dioxide (according to the prior art), the obtained surface showed a much finer metal distribution and therefore good catalytic activity in view of the aims of the present invention.

The present invention also relates to a process for preparing a material, as defined according to any one of claims 1 to 10, comprising (i) an inner part comprising or consisting of bulk silicon, (ii) an outer part comprising or consisting of a silicon-based compound, said silicon-based compound comprising silicon and a non-metal element, and (iii) clusters comprising or consisting of a transition metal, wherein said process comprises the steps of:
(a) providing a bulk silicon substrate forming a particle or layer;
(b) providing a transition metal or a source thereof to the surface of said bulk silicon substrate;
(c) converting at least a part of the surface of said bulk silicon substrate into said silicon-based compound and growing a silicon-based compound layer on said bulk silicon substrate, thereby
(d) providing a material comprising (i) an inner part comprising or consisting of bulk silicon, (ii) an outer part comprising or consisting of a silicon-based compound, said silicon-based compound comprising silicon and a non-metal element, and (iii) clusters comprising or consisting of a transition metal.

Such a process enables to provide a material according to the present invention in a reproducible manner.

### Detailed description of the invention

In one embodiment, the starting material is silicon with a flake-like morphology that can be obtained from the production of silicon wafers for the photovoltaic industry. Submicron silicon particles can also be obtained by nano-milling, e.g. by ball milling of silicon in ethanol leading to particles sizes as low as 50 nm. The invention is not limited to a specific source of silicon or processing thereof (e.g. porous silicon).

In one embodiment, the bulk silicon substrate forming a particle or layer is porous. Porous silicon (PS) is known in the field, for example see:

### https://en.wikipedia.org/wiki/Porous silicon.

In one embodiment, said porous particle or porous layer is an open foam structure.

In one embodiment, the bulk silicon substrate forming a particle or layer is not porous.

In the given embodiment, such particles present a D₅₀ of from 100 to 500 nm, typically of about 300 nm.

Due to the flake-like morphology, the silicon core or inner part has a high specific surface (compared to spherical powder with the same particle size distribution), for example of 10 to 30 m²/g, typically of about 17 m²/g according to a BET method measurement.

Advantageously, the silicon core or inner part is a non-toxic, inert and stable material. Because of it is diamond-like and bonding structure, silicon has a high mechanical strength up to 1300 °C, high hardness, and good thermal conductivity.

The term "consisting of" includes the expression "consisting essentially of". Accordingly any occurrence of the term "consisting of" means also "consisting essentially of" and/or may be replaced by "consisting essentially of".

The expression "consisting essentially of" means that other elements may be present as impurities, typically unavoidable impurities or impurities presents mainly due to the raw materials or to the process of manufacturing. In some embodiments, impurities are beneficial to certain properties of the material, for example when used as a catalyst.

The term "bulk silicon" designates the silicon element (Si).

In other words, said inner part consists of non-oxidized silicon.

Si can come from any source unless unsuitable for the present invention.

In one embodiment, Si comes from waste.

Typically, the silicon core or inner part is a silicon powder. Advantageously, the silicon core or inner part has the desired particle size and shape for the considered application.

Preferably, the silicon core or inner part has a very high purity of silicon, typically of more than 99%, typically of between 99.9 and 99.99%.

In one embodiment, such particles present surface oxidation with 10 -15 wt.% SiO₂.

In one embodiment, the silicon core or inner part comprises Ni, Al and Mg as impurities.

Clusters in the present invention comprise or consist of a transition metal and are defined as comprising of one or more atoms of a transition metal.

In one embodiment, said clusters comprise or consist of Fe.

Typically, a transition metal is an element whose atom has a partially filled d sub-shell, or which can give rise to cations with an incomplete d sub-shell according to IUPAC definition.

Typically, a transition metal is selected from the group consisting of Sc, Ti, V, Cr, Mn, Fe, Co, Ni, Cu, Zn, Y, Zr, Nb, Mo, Tc, Ru, Rh, Pd, Ag, Cd, La, Hf, Ta, W, Re, Os, Ir, Pt, Au and Hg or a mixture of thereof.

In one embodiment, said transition metal is selected from the group consisting of Fe W, Mo, Cu, Ni, Co, and V or a mixture of thereof and preferably Fe.

In one embodiment, said clusters equal 0.01 wt.% to 2 wt.% with respect to the total weight of the Silicon based-compound.

In one embodiment, said outer part comprises clusters, preferably the surface of said outer part comprises clusters. (NB: Si, not SiOₓ).

In one embodiment, said non-metal element is carbon, nitrogen, or oxygen.

In one embodiment, said silicon-based compound is selected from the group consisting of SiOₓ (typically SiO₂) SiC, SiCN, SiCO, SiN.

In one embodiment, said silicon-based compound is SiOₓ, typically SiO₂.

In one embodiment, said shell or outer part has a thickness of few nanometers, typically below 100nm, preferably below 10nm.

For example, said silicon-based compound can be crystalline or amorphous. It can also be non-stoichiometric.

In other word said inner part or core according to the invention forms a matrix, support or substrate comprising or consisting of bulk silicon (Si).

In one embodiment said clusters are in the form of particles localized at the surface of said shell or outer part, typically are particles embedded in the Si based-compound visible by Transmission electron microscopy (TEM) with a diameter of less than 20 nm, preferably less than 8 nm and even more preferably of less than 5nm.

In one embodiment, said core or inner part forms a support for the shell or outer part

Typically, said core or inner part and said shell or outer part are adjacent.

In one specific embodiment, said material comprises or consists of a Si core or inner part with Si dioxide with Fe clusters (typically forming catalytic sites).

In one embodiment, said clusters are in the form of particles having a diameter ranging from 0.1 nm to 20 nm, preferably said particles have a diameter ranging from 1 nm to 8 nm, more preferably of less than 5 nm.

In one embodiment, said core or inner part comprises an Si-alloy, preferably said Si-alloy being a transition metal and Si alloy, for example a SiFe alloy.

In one embodiment, said outer part comprises clusters, preferably the surface of said outer part comprises clusters.

In one embodiment, as a low-cost source, silicon nanoparticles produced and recovered from the manufacturing of silicon wafers may be used. In one embodiment, the silicon core or inner part is further processed, for example by drying, e.g. spray-drying, pelletizing or another process.

Another embodiment for preparation is milling of silicon or direct synthesis from the gas phase. A high surface area may also be provided by producing porous silicon (PS).

In one embodiment, prior to step (a), the process comprises a step of cleaning at least a part of the surface of the Si core or inner part, preferably the entire surface.

Typically, step (a) comprises removing metallic impurities from said bulk silicon substrate.

In one embodiment, step (a) comprises deoxidizing removing said bulk silicon substrate. Typically, depending on the method of preparation of the silicon, a deoxidation and cleaning step may be needed to remove any oxide layer and/or contaminants for activation of the silicon surface. A commonly use method is etching of silicon with a diluted HF solution to remove silicon oxides.

In one embodiment, said step (b) of providing a transition metal source to the surface of said bulk silicon substrate is performed by or comprises putting said bulk silicon substrate into contact with a solution of at least one source of one or more transition metals.

In one embodiment, said step (b) of providing a transition metal source to the surface of said bulk silicon substrate is performed by or comprises providing particles comprising a transition metal and depositing said particles on said surface of said bulk silicon substrate thereby forming said clusters.

In other word, in one embodiment, the present invention provides a step (b) of contaminating or doping at least a part of the Si surface with a transition metal.

In one embodiment, a process for preparing a material according to the present invention comprises the solidification of a silicon melt that is doped or alloyed with one or more transition metals.

In one embodiment, the solidification is performed by atomization or any other known method for rapid solidification.

In one embodiment, the process of the invention comprises exposing a Si surface, preferably a clean Si surface, to Fe or a Fe source, and attaching Fe to at least a part of the surface of the Si core or inner part. Such step may be provided by putting a Si core (particles) or layer (substrate) in a solution containing a transition metal.

In one embodiment, said process comprises a step of milling of the material to increase the surface and expose the cluster comprising or consisting of a transition metal to the surface.

In one embodiment, step b) is performed without specific heating.

In one embodiment, said step (c) comprises a thermal or chemical conversion of said bulk silicon into a silicon-based compound.

In one embodiment, said step (c) comprises or consists of a thermal treatment at a temperature of at most 800°C, preferably at most 300°C and/or of a chemical conversion.

Advantageously, the present invention implement a temperature much lower than in the prior art.

A lower temperature will reduce the risk of Oswald ripening which causes particle growth.

Typically, the heat treatment is performed in the presence of a gas forming the considered Si-based compound.

Typically, the heat treatment is perform in the presence of air, nitrogen, a carbon dioxide, methane or any mixture thereof.

In one embodiment, said step (c) comprises or consists of a thermal oxidation and/or chemical oxidation of at least a part of the surface of said bulk silicon substrate into Si dioxide and subsequent growth of a Si dioxide layer.

In one embodiment, step (c) comprises chemically oxidizing at least a part of the surface of said bulk silicon substrate into said silicon-based compound and thermally treating the oxidized material.

Advantageously, the chemical oxidation does not require high temperature, even less than heat treatment according to the present invention.

Typically, the chemical conversion is perform in the presence of a chemical oxidant of the Si inner part surface.

Such chemical oxidant are for example: hydrogen peroxide, nitric acid, ozone, etc.

In step (c), the silicon-based compound is grown on the Si core or inner part surface physically and/or chemically.

In one embodiment, step (c) is performed under conditions oxidizing the silicon surface into a Si dioxide layer.

In one embodiment, the present invention relates to a process comprising a doping of bulk Si nanoparticles with a transition metal, typically Fe, followed by chemical oxidation, possibly combined, sequentially, with a heat treatment at a temperature of at most 800°C, preferably at most 300°C.

In one embodiment, the silicon surface is doped with the desired transition metal, for example Fe, and the chemical oxidation at the same time (e.g. by using a FeCI / H₂O₂ solution).

In one embodiment, the silicon surface is doped with the desired transition metal, for example Fe. This can be performed by bringing the bulk silicon in contact with an aqueous solution of the desired transition metal ions.

In one embodiment, said doping is performed by direct deposition from a gas phase and/or precursors as a source of said transition metal, e.g. by ALD, or by use of surface organometallic chemistry.

In one embodiment, the conversion of the surface (or part thereof) into a Si-based compound is followed under the same or similar condition by growth of the Si-based compound on the Si core or inner part surface thereby forming said Si-based compound shell or outer part.

In one embodiment, silicon oxide can be grown by thermal and/or chemical oxidation. During step (c), the transition metal is integrated into the silicon oxide layer.

Accordingly, a very thin outer part or shell of Si-based compound is doped with a transition metal located inside of said shell or outer part.

Accordingly, in one embodiment, said clusters are preferably located inside said shell or outer part.

In one embodiment, said outer part comprises clusters on the external surface of the outer part.

In one specific embodiment, said process provides a substrate consisting of Si as a core (pure Si - with little contamination) forming a layer of a transition metal on the Si core, and converting at least a part of the surface of said silicon core (or Si substrate) into silicon oxide and growing a silicon oxide layer on the surface of the Si core thereby forming a SiOₓ shell with said transition metal forming clusters dispersed in the SiOₓ shell.

The present invention relates to a method for catalyzing a chemical reaction, said method comprising catalyzing said reaction by a material (used as a catalyst) as defined according to the present invention or obtainable according to a process as defined according to the present invention.

In one embodiment, said reaction is a natural gas conversion, typically is a methane conversion into petrochemicals (ethylene, benzene) and hydrogen.

Advantageously, a material (catalyst), according to the present invention, provides notably high selectivity towards hydrocarbons in gas conversion and/or low tendency for coke formation.

Advantageously, a material (catalyst) according to the present invention provides a catalyst for the oil, gas or chemical industry, typically for the petro-chemical industry.

Advantageously, a material (catalyst), according to the present invention, provides a catalyst for the conversion of natural gas (methane) to higher value products such as aromatic compounds, for example benzene or naphthalene.

Advantageously, a material (catalyst), according to the present invention, provides a catalyst for the conversion methane into ethylene.

Advantageously, a material (catalyst) according to the present invention provides a catalyst for plastic pyrolysis.

Typically, said clusters can act as catalytically active sites, typically for one or more of the above reactions.

With regard to the process for the conversion of methane into petrochemicals (ethylene, benzene) and hydrogen, this reaction is depicted below:

2 CH₄ → C₂H₄ + 2 H₂ or 6CH4 →C₆H₆ + 9 H₂

The present invention relates to a method for conversion of natural gas into petrochemicals and hydrogen under non-oxidative conditions, wherein said material as defined according to the present invention or prepared by a process as defined according to the present inventi, is implemented as a catalyst in conversion of natural gas into petrochemicals and hydrogen under non-oxidative conditions.The reaction, it is typically carried out under non oxidative conditions (in absence of oxygen) in a reactor comprising a catalyst, which is active in the conversion of the methane- containing gas stream. The methane-containing gas stream that is fed to the reactor comprises more than 50 % vol. methane, preferably more than 70 % vol. methane and more preferably of from 75 % vol. to 100 % vol. methane. The balance of the methane- containing gas may be other alkanes, for example, ethane, propane. The methane-containing gas stream may be natural gas which is a naturally occurring hydrocarbon gas mixture consisting primarily of methane, with up to about 30 % vol. concentration of other hydrocarbons (usually mainly ethane and propane), as well as small amounts of other impurities such as carbon dioxide, nitrogen and others. The conversion of a methane-containing gas stream is carried out at a weight hourly space velocity of from 0.1 to 100 h⁻¹, a pressure of from 0.5 to 50 bar and a temperature or from 500 to 1100 °C. More preferably, the conversion is carried out at weight hourly space velocity (chemistry) (WHSV) of from 0.5 to 50 h⁻¹, a pressure of from 0.5 to 10 bar and a temperature of from 750 to 1000 °C. Even more preferably, the conversion is carried out at WHSV of from 1 to 30 h-1, a pressure of from 0.5 to 8 bar and a temperature of from 800 to 950 °C. Various co-feeds such as CO2, steam or hydrogen or mixtures thereof that react with coke precursors or prevent their formation during methane aromatization can be added at levels of < 30 % vol. to the methane-containing feed to improve the performance of the catalyst.

In an embodiment, the catalyst is pre-treated before the reaction with a stream containing CO, CO₂, C₂H₄, C₂H₆, H₂O, C₃₊ hydrocarbon mixture containing at least 10 wt% of acyclic hydrocarbons or a mixture of thereof at a temperature between 450°C and 750°C, WHSV -0.1 -100 h⁻¹, pressure between 0.1 and 10 barg.

In an embodiment, the pre-treatment will be performed with CH₄ -containing stream at WHSV between 0.1 -1.5 h⁻¹, temperature range 650°C -850°C and pressure 1 - 10 barg.

In a preferred embodiment, the reactor used at step iii) can comprise a tubular reactor, a continuous flow reactor, a riser reactor, a reformer reactor, a fixed bed reactor, a shock tube reactor, a multi-tubular reactor, a membrane reactor, a dual flow reactor, a gauze reactor, a fluidized bed reactor, a moving bed reactor, a continuous stirred-tank reactor (CSTR), a plug flow reactor (PFR), a microchannel reactor, a modular reactor, a modular microchannel reactor, a honeycombed monolithic reactor, a honeycombed wall filter monolithic reactor, and the like, or combinations thereof. In an embodiment, the reactor can comprise a reformer reactor, a fixed bed reactor, a fluidized bed reactor, a moving bed reactor, and the like, or combinations thereof.

In the present invention, the term "a" means "one or more". For example, clusters comprising or consisting of a transition metal means clusters comprising or consisting of one or more transition metals.

### Example

### Preparation of a material according to the invention

In a first step to remove metallic impurities, the silicon material, for example dry and foreign (abrasive) particle free silicon kerf from the silicon wafer production, is washed by mixing 1 L of 5 wt.% HCI with 200 g of Si powder and agitating the solution for 10 minutes by stirring.

The silicon particles are separated by membrane filtration or centrifugation from the solution and then re-dispersed in 1 L of 5 wt.% HF for deoxidation and again separated by before mentioned methods.

Subsequently, the silicon surface is doped with Fe by mixing the silicon particles in 1 L DI water containing 10 g of iron nitrate.

Following a separation step, the silicon powder is oxidized for 20 minutes by mixing it with 1 L of 3 wt.% hydrogen peroxide.

After separation the silicon material, now consisting of a silicon core and a shell of SiFexOv, is dried at 200 °C for 2 hours.

The obtained silicon material comprises (i) an inner part comprising or consisting of bulk silicon, (ii) an outer part (in the present case SiFexOv) comprising or consisting of a silicon-based compound, said silicon-based compound comprising of silicon and a non-metal element (in the present example: Oxygen), and (iii) clusters comprising or consisting of a transition metal (in the present example: Fe).

## Claims

1. A material comprising (i) an inner part comprising or consisting of bulk silicon, (ii) an outer part comprising or consisting of a silicon-based compound, said silicon-based compound comprising of silicon and a non-metal element, and (iii) clusters comprising or consisting of a transition metal.

2. The material according to claim 1, wherein said non-metal element is carbon, nitrogen, or oxygen.

3. The material according to claim 1 or 2, wherein said transition metal is selected from the group consisting of Fe, W, Mo, Cu, Ni, Co, and V or a mixture of thereof, and preferably Fe.

4. The material according to any one of claims 1 to 3, wherein said clusters equal 0.01 wt.% to 2 wt.% with respect to the total weight of the Silicon based-compound.

5. The material according to any one of claims 1 to 4, wherein said clusters are in the form of particles localized at the surface of said shell or outer part, typically are particles embedded in the Si based-compound visible by Transmission electron microscopy (TEM) with a diameter of less than 20 nm, preferably less than 8 nm and even more preferably of less than 5nm.

6. The material according to any one of claims 1 to 5, wherein said clusters are in the form of particles having a diameter ranging from 0.1 nm to 20 nm, preferably said particles have a diameter ranging from 1 nm to 8 nm, more preferably of less than 5 nm.

7. The material according to any one of claims 1 to 6, wherein said core or inner part comprises an Si-alloy, preferably said Si-alloy being a transition metal and Si alloy, for example a SiFe alloy.

8. The material according to any one of claims 1 to 7, wherein said outer part comprises clusters, preferably the surface of said outer part comprises clusters.

9. The material according to any one of claims 1 to 8, wherein said material is a catalyst.

10. A process for preparing a material, as defined according to any one of claims 1 to 9, comprising (i) an inner part comprising or consisting of bulk silicon, (ii) an outer part comprising or consisting of a silicon-based compound, said silicon-based compound comprising silicon and a non-metal element, and (iii) clusters comprising or consisting of a transition metal, wherein said process comprises the steps of:
(a) providing a bulk silicon substrate forming a particle or layer;
(b) providing a transition metal or a source thereof to the surface of said bulk silicon substrate;
(c) converting at least a part of the surface of said bulk silicon substrate into said silicon-based compound and growing a silicon-based compound layer on said bulk silicon substrate, thereby
(d) providing a material comprising (i) an inner part comprising or consisting of bulk silicon, (ii) an outer part comprising or consisting of a silicon-based compound, said silicon-based compound comprising silicon and a non-metal element, and (iii) clusters comprising or consisting of a transition metal.

11. The method according to claim 10, wherein said step (b) of providing a transition metal source to the surface of said bulk silicon substrate is performed by or comprises putting said bulk silicon substrate into contact with a solution of at least one source of one or more transition metals.

12. The method according to claim 10, wherein said step (b) of providing a transition metal source to the surface of said bulk silicon substrate is performed by or comprises providing particles comprising a transition metal and depositing said particles on said surface of said bulk silicon substrate thereby forming said clusters.

13. The method according to any one of claims 10 to 12, wherein said step (c) comprises or consists of a thermal treatment at a temperature of at most 800°C, preferably at most 300°C and/or of a chemical conversion.

14. The method according to any one of claims 10 to 13, wherein said step (c) comprises or consists of a thermal oxidation and/or chemical oxidation of at least a part of the surface of said bulk silicon substrate into Si dioxide and subsequent growth of a Si dioxide layer.

15. A method for conversion of natural gas into petrochemicals and hydrogen under non-oxidative conditions, wherein said material as defined according to any one of claims 1 to 9 or prepared by a process as defined in any one of claims 10 to 14, is implemented as a catalyst in conversion of natural gas into petrochemicals and hydrogen under non-oxidative conditions.
